# EUROPEAN PATENT SPECIFICATION

(11) **EP 1 137 432 B1**
(45) Date of publication and mention of the grant of the patent: **14.04.2004**
(21) Application number: 99961896.0
(22) Date of filing: 01.12.1999
(51) Int. Cl.: A61K 38/48, A61P 7/02

(54) **USE OF PROTEIN C FOR THE TREATMENT OF THROMBOCYTOPENIC PURPURA AND HEMOLYTIC UREMIC SYNDROME**
VERWENDUNG VON PROTEIN C ZUR BEHANDLUNG VON THROMBOZYTOPENISCHER PURPURA UND HÄMOLYTISCHES URÄMISCHES SYNDROM
UTILISATION DE LA PROTEIN C POUR LE TRAITEMENT DU PURPURA THROMBOPENIQUE ET DU SYNDROME HEMOLYTIQUE ET UREMIQUE

(30) Priority: 10.12.1998 US 111770 P
(43) Date of publication of application: 04.10.2001
(73) Proprietor: ELI LILLY AND COMPANY, Indianapolis, Indiana 46285 (US)
(72) Inventor: FISHER, Charles, Jack, Carmel, IN 46033 (US); YAN, Sau-Chi, Betty, Indianapolis, IN 46240 (US)
(74) Representative: Denholm, Anna Marie
(86) International application number: PCT/US1999/028433
(87) International publication number: WO 2000/033867

(56) References cited:
- EP-A- 0 875 252
- WADA H ET AL.: "Poor Outcome in Disseminated Intravascular Coagulation or Thrombotic Thrombocytopenic Purpura Patients with Severe Vascular Cell Injuries " AMERICAN JOURNAL OF HEMATOLOGY, vol. 58, no. 3, July 1998 (1998-07), pages 189-194, XP000891310

## Description

This invention relates to medical science particularly the treatment of thrombotic thrombocytopenic purpura and hemolytic uremic syndrome with protein C.

Protein C is a vitamin K dependent serine protease and naturally occurring anticoagulant that plays a role in the regulation of hemostasis by inactivating Factors Va and VIIIa in the coagulation cascade. Human protein C circulates as a 2-chain zymogen, but functions at the endothelial and platelet surface following conversion to activated protein C (aPC) by limited proteolysis with thrombin in complex with the cell surface membrane protein, thrombomodulin.

In conjunction with other proteins, aPC functions as perhaps the most important down-regulator of blood coagulation resulting in protection against thrombosis. In addition to its anti-coagulation functions, aPC has anti-inflammatory effects through its inhibition of cytokine generation (e.g. TNF and IL-1) and also exerts profibrinolytic properties, such as the inhibition of PAI-1, that facilitate clot lysis. Thus, the protein C enzyme system represents a major physiological mechanism of anti-coagulation, anti-inflammation, and fibrinolysis.

EP 0 875 252 A relates to pharmaceutical formulations of activated protein C and the use of activated protein C in medicaments for the treatment of disease states involving intravascular coagulation.

Thrombotic thrombocytopenic purpura (TTP) and hemolytic uremic syndrome (HUS) are defined as thrombotic microangiopathies characterized by occlusion of arterioles and capillaries by microthrombi, thrombocytopenia, impairment of neurologic function and progressive renal failure. TTP has been defined as a multisystem disease characterized by fever, fluctuating central nervous system abnormalities, renal failure, microangiopathic hemolytic anemia and thrombocytopenia. HUS is characterized by intravascular hemolytic anemia associated with fragmented red cells; thrombocytopenia; and end-organ damage with either (a) histological evidence of a thrombotic microangiopathic process (most commonly in the kidneys), or (b) clinical evidence of such damage in the absence of any other disease or likely cause [Neild, G., Kidney International 53(Suppl. 64): S-45-S-49, 1998].

Contributing factors to the pathophysiology of TTP/HUS are believed to be endothelial cell damage and primary platelet aggregation [Moake, Seminars in Hematology, 34(2): 83-89, 1997]. Endothelial damage leads to release of unusually large von Willebrand factor (UL vWF) multimers, which in turn leads to platelet aggregation. Fibrin thrombi accumulate on the platelet thrombi as a result of depression of the body's fibrinolytic system. In HUS and TTP elevated levels of plasminogen activator inhibitor (PAI-1) and decreased levels of protein C activity have been found.

Wada *et al*, American Journal of Hematology, 58:189-194, (1998) describes experimental research comprising measuring various hemostatic and vascular endothelial cell markers in patients with disseminated intravascular coagulation (DIC), non-DIC, or thrombotic thrombocytopenic purpura (TTP) and in healthy volunteers to examine the relationships between hemostatic abnormalities or vascular endothelial cell injuries and the patients' outcomes.

TPP has been associated with bacterial infections of the *Bartonella* sp., as well as with HIV and visceral Kaposi's sarcoma [Avery *et al*, American Journal of Hematology, 58:148-149, 1998]. TTP has also been associated with the use of numerous drugs, for example, ticlopidine, FK506, high-dose corticosteroids, tamoxiphen, or cyclosporin A [Gordon *et al.,* Seminars in Hematology, 34(2): 140-147, 1997].

HUS has been associated with numerous bacterial infections, for example, E. coli O157:H7 strain, Shigella, Pneumococci, hemolytic Streptococci, or Yersinia. HUS has also been associated with viral infections, for example, HIV, Coxsackie, or adenovirus. In addition, HUS has been associated with the use of numerous drugs similar to those listed above for TPP [Gordon et al., 1997]. Both TTP and HUS have been associated with complications during pregnancy [Egerman *et al*., Am J Obstet Gynecol, 175: 950-956, 1996].

The clinical findings of thrombotic microangiopathies include microangiopathic hemolytic anemia (MAHA), acute renal failure, thrombocytopenia and in TTP, acute neurologic changes. Left untreated, TTP and HUS have a poor prognosis with mortality rates reaching 90%. Surviving patients often develop end-stage renal disease. The only treatment method with a favorable outcome has been plasma exchange [PE] with fresh frozen plasma [Hollenbeck *et al*., Nephrol Dial Transplant 13: 76-81, 1998]. However, deaths still occur and many patients suffer long term complications even if treated with PE. Therefore, a need exists for a more effective treatment of TTP and/or HUS.

The present invention is the first to describe the use of protein C in the manufacture of a medicament for treating a patient suffering from TTP and/or HUS. Protein C, with its anticoagulant and profibrinolytic activities along with its ability to inactivate PAI-1, is useful in the manufacture of a medicament for the treatment of the occlusion of arterioles and capillaries by microthrombi that occur in patients with TTP and HUS.

The present invention provides the use of protein C in the manufacture of a medicament for treating a patient suffering from thrombotic thrombocytopenic purpura (TTP).

In another embodiment, the present invention provides the use of a protein C in the manufacture of a medicament for treating a patient suffering from hemolytic uremic syndrome (HUS).

In another embodiment, the present invention provides the use of activated protein C in the manufacture of a medicament for treating a patient suffering from thrombotic thrombocytopenic purpura (TTP), wherein said medicament is adapted for administration in an amount sufficient to provide an activated protein C plasma level of about 2 ng/ml to 300 ng/ml.

In still another embodiment, the present invention provides the use of activated protein C in the manufacture of a medicament for treating a patient suffering from hemolytic uremic syndrome (HUS), wherein said medicament is adapted for administration in an amount sufficient to provide an activated protein C plasma level of about 2 ng/ml to 300 ng/ml.

For purposes of the present invention, as disclosed and claimed herein, the following terms are as defined below.

Protein C refers to a vitamin K dependent serine protease with anticoagulant, anti-inflammatory, and profibrinolytic properties which includes, but is not limited to, plasma derived and recombinant produced protein C. Protein C includes and is preferably human protein C although protein C may also include other species or derivatives having protein C proteolytic, amidolytic, esterolytic, and biological (anticoagulant, profibrinolytic, and anti-inflammatory) activities. Examples of protein C derivatives are described by Gerlitz, *et al*., U.S. Patent No. 5,453,373, and Foster, *et al*., U.S. Patent No. 5,516,650.

Zymogen - an enzymatically inactive precursor of a proteolytic enzyme. Protein C zymogen, as used herein, refers to secreted, inactive forms, whether one chain or two chains, of protein C.

Activated protein C or aPC refers to protein C zymogen which has been converted by limited proteolysis to its activated form. aPC includes and is preferably human protein C although aPC may also include other species or derivatives having protein C proteolytic, amidolytic, esterolytic, and biological (anticoagulant or profibrinolytic) activities. Examples of protein C derivatives are noted above in the description of protein C.

HPC - human protein C zymogen.

r-hPC - recombinant human protein C zymogen.

r-aPC - recombinant human activated protein C produced by activating r-hPC *in vitro* or by direct secretion of the activated form of protein C from procaryotic cells, eukaryotic cells, and transgenic animals or plants, including, for example, secretion from human kidney 293 cells as a zymogen then purified and activated by techniques well known to the skilled artisan and demonstrated in Yan, U.S. Patent No. 4,981,952, and Cottingham, WO97/20043.

Plasma derived activated protein C - activated protein C produced by activating plasma HPC as described in Eibl, U.S. Patent No. 5,478,558.

Continuous infusion - continuing substantially uninterrupted the introduction of a solution into a vein for a specified period of time.

Bolus injection - the injection of a drug in a defined quantity (called a bolus) over a period of time up to about 120 minutes.

Suitable for administration - a lyophilized formulation or solution that is appropriate to be given as a therapeutic agent.

Unit dosage form - refers to physically discrete units suitable as unitary dosages for human subjects, each unit containing a predetermined quantity of active material calculated to produce the desired therapeutic effect, in association with a suitable pharmaceutical excipient.

Pharmaceutically effective amount - represents an amount of a compound of the invention that is capable of inhibiting sepsis in humans. The particular dose of the compound administered according to this invention will, of course, be determined by the attending physician evaluating the particular circumstances surrounding the case.

The present invention provides the use of protein C in the manufacture of a medicament for treating a patient suffering from thrombotic thrombocytopenic purpura (TTP) and/or hemolytic uremic syndrome (HUS). TTP has been defined as a multisystem disease characterized by fever, fluctuating central nervous system abnormalities, renal failure, microangiopathic hemolytic anemia and thrombocytopenia. HUS is characterized by intravascular hemolytic anemia associated with fragmented red cells; thrombocytopenia; and end-organ damage with either (a) histological evidence of a thrombotic microangiopathic process (most commonly in the kidneys), or (b) clinical evidence of such damage in the absence of any other disease or likely cause. Protein C, with its anticoagulant and profibrinolytic activities along with its ability to inactivate PAI-1, is useful in the manufacture of a medicament for the treatment of the occlusion of arterioles and capillaries by microthrombi that occur in patients with TTP and HUS.

The protein C administered according to this invention may be generated and/or isolated by any means known in the art or as described in U.S. Patent No. 4,981,952, and U.S. Patent No. 5,550,036. For example, the invention provides a method for producing and secreting full-length, soluble protein C, or biologically active polypeptide variants of protein C from a cell which comprises (a) constructing a vector comprising DNA encoding protein C; (b) transfecting the cell with the vector; and (c) culturing the cell so transfected in culture medium under conditions such that full length soluble protein C or biologically active polypeptide variants of protein C, is secreted. Further, the cell is a eukaryotic cell, e.g. mammalian cell such as Syrian hamster AV12 cell, human embryonic 293 cell, or Baby Hamster Kidney cell.

The protein C used in the manufacture of a medicament for the treatment of TTP/HUS can be formulated according to known methods to prepare pharmaceutically useful compositions. For example, a desired formulation would be one that is a stable lyophilized product of high purity comprising a bulking agent such as sucrose, a salt such as sodium chloride, a buffer such as sodium citrate and protein C or aPC.

The protein C will be administered parenterally to ensure its delivery into the bloodstream in an effective form by injecting the appropriate dose as continuous infusion for about 1 hour to about 240 hours.

Those skilled in the art can readily optimize pharmaceutically effective dosages and administration regimens for therapeutic compositions comprising protein C, as determined by good medical practice and the clinical condition of the individual patient. Generally, the amount of protein C administered will be from about 5.0 µg/kg/hr to about 250 µg/kg/hr. Preferably, the protein C used in the treatment of TTP/HUS is activated protein C. The amount of aPC administered will be from about 1.0 µg/kg/hr to about 96 µg/kg/hr. More preferably the amount of aPC administered will be about 1.0 µg/kg/hr to about 50 µg/kg/hr. While more preferably the amount of aPC administered will be about 1.0 µg/kg/hr to about 35 µg/kg/hr. Even more preferably the amount of aPC administered will be about 5.0 µg/kg/hr to about 30 µg/kg/hr. Yet even more preferably the amount of aPC administered will be about 15 µg/kg/hr to 30 µg/kg/hr. Still even more preferably the amount of aPC administered will be about 20 µg/kg/hr to 30 µg/kg/hr. The most preferable amount of aPC administered will be about 24 µg/kg/hr. The appropriate dose of aPC administered will result in a reduction of the occlusions of arterioles and capillaries by microthrombi that occur in patients with TTP and HUS.

The plasma ranges obtained from the amount of aPC administered will be about 2 ng/ml to about 300 ng/ml. The preferred plasma ranges are from about 2 ng/ml to 200 ng/ml. Most preferably, plasma ranges are from about 30 ng/ml to about 150 ng/ml and still more preferably about 100 ng/ml.

Alternatively, the aPC will be administered by injecting one third of the appropriate dose per hour as a bolus injection followed by the remaining two thirds of the hourly dose as continuous infusion for one hour followed by continuous infusion of the appropriate dose for twenty-three hours which results in the appropriate dose administered over 24 hours. In addition, the bolus injection will be administered via an intravenous bag drip pump or syringe pump at about 2 times the normal rate for about 10 to 20 minutes followed by about 1.5 times the normal rate for about 40 to 50 minutes. The normal rate i.e. that rate which has been determined to administer the appropriate dose level of the therapeutic agent per time period, is then continued for up to 240 hours.

The use of protein C in the manufacture of a medicament for the treatment of TTP/HUS as presented in the present invention will provide a needed therapy for potentially serious and debilitating disorders. The use of protein C is efficacious and avoids the complications such as toxicity and general side effects of the currently available therapy of plasma exchange [PE] with fresh frozen plasma or other currently available anti-coagulant agents.

The following examples are provided merely to further illustrate the present invention. The scope of the invention shall not be construed as merely consisting of the following examples.

### Preparation 1

### Preparation of Human Protein C

Recombinant human protein C (r-hPC) was produced in Human Kidney 293 cells by techniques well known to the skilled artisan such as those set forth in Yan, U.S. Patent No. 4,981,952. The gene encoding human protein C is disclosed and claimed in Bang, et al., U.S. Patent No. 4,775,624. The plasmid used to express human protein C in 293 cells was plasmid pLPC which is disclosed in Bang, *et al*., U.S. Patent No. 4,992,373. The construction of plasmid pLPC is also described in European Patent Publication No. 0 445 939, and in Grinnell, *et al*., 1987, Bio/Technology 5:1189-1192. Briefly, the plasmid was transfected into 293 cells, then stable transformants were identified, subcultured and grown in serum-free media. After fermentation, cell-free medium was obtained by microfiltration.

The human protein C was separated from the culture fluid by an adaptation of the techniques of Yan, U.S. Patent No. 4,981,952. The clarified medium was made 4 mM in EDTA before it was absorbed to an anion exchange resin (Fast-Flow Q, Pharmacia). After washing with 4 column volumes of 20 mM Tris, 200 mM NaCl, pH 7.4 and 2 column volumes of 20 mM Tris, 150 mM NaCl, pH 7.4, the bound recombinant human protein C zymogen was eluted with 20 mM Tris, 150 mM NaCl, 10 mM CaCl₂, pH 7.4. The eluted protein was greater than 95% pure after elution as judged by SDS-polyacrylamide gel electrophoresis.

Further purification of the protein was accomplished by making the protein 3 M in NaCl followed by adsorption to a hydrophobic interaction resin (Toyopearl Phenyl 650 M, TosoHaas) equilibrated in 20 mM Tris, 3 M NaCl, 10 mM CaCl₂, pH 7.4. After washing with 2 column volumes of equilibration buffer without CaCl₂, the recombinant human protein C was eluted with 20 mM Tris, pH 7.4.

The eluted protein was prepared for activation by removal of residual calcium. The recombinant human protein C was passed over a metal affinity column (Chelex-100, Bio-Rad) to remove calcium and again bound to an anion exchanger (Fast Flow Q, Pharmacia). Both of these columns were arranged in series and equilibrated in 20 mM Tris, 150 mM NaCl, 5 mM EDTA, pH 7.4. Following loading of the protein, the Chelex-100 column was washed with one column volume of the same buffer before disconnecting it from the series. The anion exchange column was washed with 3 column volumes of equilibration buffer before eluting the protein with 0.4 M NaCl, 20 mM Tris-acetate, pH 6.5. Protein concentrations of recombinant human protein C and recombinant activated protein C solutions were measured by UV 280 nm extinction E^{0.1%}=1.81 or 1.85, respectively.

### Preparation 2

### Activation of Recombinant Human Protein C

Bovine thrombin was coupled to Activated CH-Sepharose 4B (Pharmacia) in the presence of 50 mM HEPES, pH 7.5 at 4°C. The coupling reaction was done on resin already packed into a column using approximately 5000 units thrombin/mL resin. The thrombin solution was circulated through the column for approximately 3 hours before adding 2-amino-ethanol (MEA) to a concentration of 0.6 mL/L of circulating solution. The MEA-containing solution was circulated for an additional 10-12 hours to assure complete blockage of the unreacted amines on the resin. Following blocking, the thrombin-coupled resin was washed with 10 column volumes of 1 M NaCl, 20 mM Tris, pH 6.5 to remove all non-specifically bound protein, and was used in activation reactions after equilibrating in activation buffer.

Purified r-hPC was made 5 mM in EDTA (to chelate any residual calcium) and diluted to a concentration of 2 mg/mL with 20 mM Tris, pH 7.4 or 20 mM Tris-acetate, pH 6.5. This material was passed through a thrombin column equilibrated at 37°C with 50 mM NaCl and either 20 mM Tris pH 7.4 or 20 mM Tris-acetate pH 6.5. The flow rate was adjusted to allow for approximately 20 min. of contact time between the r-hPC and thrombin resin. The effluent was collected and immediately assayed for amidolytic activity. If the material did not have a specific activity (amidolytic) comparable to an established standard of protein C, it was recycled over the thrombin column to activate the r-hPC to completion. This was followed by 1:1 dilution of the material with 20 mM buffer as above, with a pH of either 7.4 or 6.5 to keep the protein C at lower concentrations while it awaited the next processing step.

Removal of leached thrombin from the protein C material was accomplished by binding the protein C to an anion exchange resin (Fast Flow Q, Pharmacia) equilibrated in activation buffer (either 20 mM Tris, pH 7.4 or 20 mM Tris-acetate, pH 6.5) with 150 mM NaCl. Thrombin does not interact with the anion exchange resin under these conditions, but passes through the column into the sample application effluent. Once the protein C is loaded onto the column, a 2-6 column volume wash with 20 mM equilibration buffer is done before eluting the bound protein C with a step elution using 0.4 M NaCl in either 5 mM Tris-acetate, pH 6.5 or 20 mM Tris, pH 7.4. Higher volume washes of the column facilitated more complete removal of the dodecapeptide. The material eluted from this column was stored either in a frozen solution (-20°C) or as a lyophilized powder.

The anticoagulant activity of activated protein C was determined by measuring the prolongation of the clotting time in the activated partial thromboplastin time (APTT) clotting assay. A standard curve was prepared in dilution buffer (1 mg/mL radioimmunoassay grade bovine serum albumin [BSA], 20 mM Tris, pH 7.4, 150 mM NaCl, 0.02% NaN₃) ranging in protein C concentration from 125-1000 ng/mL, while samples were prepared at several dilutions in this concentration range. To each sample cuvette, 50 µL of cold horse plasma and 50 µL of reconstituted activated partial thromboplastin time reagent (APTT Reagent, Sigma) were added and incubated at 37°C for 5 min. After incubation, 50 µL of the appropriate samples or standards were added to each cuvette. Dilution buffer was used in place of sample or standard to determine basal clotting time. The timer of the fibrometer (CoA Screener Hemostasis Analyzer, American Labor) was started immediately after the addition of 50 µL 37°C 30 mM CaCl₂ to each sample or standard. Activated protein C concentration in samples are calculated from the linear regression equation of the standard curve. Clotting times reported here are the average of a minimum of three replicates, including standard curve samples.

The above descriptions enable one with appropriate skill in the art to prepare protein C for utilization in the manufacture of a medicament for the treatment of thrombotic thrombocytopenic purpura and hemolytic uremic syndrome.

### Preparation 3

### Formulation of Activated Protein C

A stable lyophilized formulation of activated protein C was prepared by a process which comprises lyophilizing a solution comprising about 2.5 mg/mL activated protein C, about 15 mg/mL sucrose, about 20 mg/mL NaCl, and a sodium citrate buffer having a pH greater than 5.5 but less than 6.5. Additionally, the stable lyophilized formulation of activated protein C comprises lyophilizing a solution comprising about 5 mg/mL activated protein C, about 30 mg/mL sucrose, about 38 mg/mL NaCl, and a citrate buffer having a pH greater than 5.5 but less than 6.5.

The ratio of protein C:salt:bulking agent (w:w:w) is an important factor in a formulation suitable for the freeze drying process. The ratio varies depending on the concentration of protein C, salt selection and concentration and bulking agent selection and concentration. Particularly, a ratio of about 1 part activated protein C to about 7.6 parts salt to about 6 parts bulking agent is preferred.

A unit dosage formulation of activated protein C suitable for administration by continuous infusion was prepared by mixing activated protein C, NaCl, sucrose, and sodium citrate buffer. After mixing, 4 mL of the solution was transferred to a unit dosage receptacle and lyophilized. The unit dosage receptacle containing about 5 mg to about 20 mg of activated protein C, suitable for administering a dosage of about 0.01 mg/kg/hr to about 0.05 mg/kg/hr to patients in need thereof, was sealed and stored until use.

### Example 1

### A Double Blind Placebo-Controlled Randomized Trial of Recombinant Activated protein C (r-aPC) in the treatment of Thrombotic Microangiopathy.

Hemolytic-Uremic Syndrome (HUS) and Thrombotic Thrombocytopenic Purpura (TTP) are two types of thrombotic microangiopathy that have endothelial damage as the inciting event. In these disease states, the endothelium is by either verotoxins from infectious organisms such as E.coli O157:H7 and Shigella, drugs such as FK506 and ticlopidine, or autoantibodies. Endothelial damage leads to release of unusually large von Willebrand factor multimers, which in turn leads to platelet aggregation. Fibrin thrombi accumulate on the platelet thrombi as a result of depression of the body's fibrinolytic system.

In HUS and TTP, elevated levels of plasminogen activator inhibitor (PAI-1) and decreased levels of protein C activity have been found. The clinical findings of thrombotic microangiopathies include microangiopathic hemolytic anemia (MAHA), acute renal failure, thrombocytopenia, and in TTP, acute neurologic changes.

The primary objective of this trial is to show that infusion of r-aPC leads to a statistically significant reduction in time to remission of the illness compared with placebo. Secondary objectives are to show that infusion of r-aPC leads to statistically significant reductions in the number of days with renal failure, number of transfusions required, and number of convulsive episodes. The primary safety objective of this trial is to show that r-aPC does not lead to a statistically significant increase in the number of clinically significant bleeding events when compared to placebo.

Inclusion criteria for patients considered for entry into the trial is evidence of a thrombotic microangiopathy as defined by the presence of: 1) Thrombocytopenia defined as a platelet count <80X 10⁹ cells/Liter; 2) Microangiopathic Hemolytic anemia (MAHA) defined as having >2 schistocytes per visual field on peripheral blood smear, a negative direct and indirect Coombs' test, and a Hgb of <10g/dl; 3) acute renal insufficiency defined as a doubling in the patient's baseline serum creatinine or the presence of oliguria defined as a urine output <0.5 ml/kg/hr; and, 4) normal coagulation times (PT, PTT and fibrinogen). Patients are excluded from the trial if they are receiving an anticoagulant or an investigational agent. Patients with active bleeding from the respiratory or gastrointestinal tract are excluded from the study.

Patients who meet all inclusion criteria and no exclusion criteria are randomized to receive either placebo or a 96 hour infusion of r-aPC at a dose up to 48µg/kg/hr, which has previously been shown to raise the aPTT to 2X baseline. The following data is collected during the study: Hgb, platelet count, serum creatinine, 24 urine output, number of convulsive episodes, and the number of packed red blood cells transfused. The primary endpoint analyzed, i.e. time to remission of the illness, is defined by a persistent platelet count for 2 days in the absence of platelet transfusions of >100 X 10⁹ cells/Liter.

Therefore, therapy with r-aPC, with its anticoagulant and profibrinolytic activities along with its ability to inactivate PAI-1, is useful in the manufacture of a medicament for the treatment of the thrombotic microangiopathy that occur in patients with TTP and HUS.

## Claims

1. The use of protein C in the manufacture of a medicament for treating a patient suffering from thrombotic thrombocytopenic purpura or hemolytic uremic syndrome.

2. The use of Claim 1 wherein the protein C is human protein C zymogen.

3. The use of Claim 1 wherein the protein C is human activated protein C.

4. The use according to Claim 3, wherein said medicament is adapted for administration in an amount sufficient to provide about 1 µg/kg/hr to about 50 µg/kg/hr of human activated protein C.

5. The use of Claim 4, wherein the human activated protein C is to be administered by continuous infusion for about 1 to about 240 hours.

6. The use of Claim 1 wherein the protein C is activated protein C, and wherein said medicament is adapted for administration in an amount sufficient to provide an activated protein C plasma level of about 2 ng/ml to about 300 ng/ml.

7. The use of Claim 6 wherein the activated protein C is adapted for administration as a bolus injection.

8. The use of Claim 6 wherein the activated protein C is adapted for administation by continuous infusion for about 1 to about 240 hours.

9. The use of Claim 6 wherein the activated protein C is adapted for administation first as a bolus then as a continuous infusion.

10. The use of Claim 9 wherein one third of the activated protein C required to achieve activated protein C plasma levels in the range of about 2 ng/ml to about 300 ng/ml is to be administered in a bolus injection and the remaining two thirds of the activated protein C is to be subsequently administered by continuous infusion.

## Patentansprüche

1. Verwendung von Protein C bei der Herstellung eines Arzneimittels zur Behandlung eines Patienten, der an thrombotischer thrombozytopenischer Purpura oder an hämolytischem Urämiesyndrom leidet.

2. Verwendung nach Anspruch 1, worin das Protein C humanes Protein C Zymogen ist.

3. Verwendung nach Anspruch 1, worin das Protein C humanes aktiviertes Protein C ist.

4. Verwendung nach Anspruch 3, worin das Arzneimittel angepasst ist zur Verabreichung in einer Menge, die zur Bildung von etwa 1 µg/kg/h bis etwa 50 µg/kg/h an humanem aktiviertem Protein C ausreicht.

5. Verwendung nach Anspruch 4, worin das humane aktivierte Protein C durch kontinuierliche Infusion während etwa 1 bis etwa 240 h verabreicht werden soll.

6. Verwendung nach Anspruch 1, worin das Protein C aktiviertes Protein C ist und worin das Arzneimittel angepasst ist zur Verabreichung in einer Menge, die zur Bildung eines aktivierten Protein C Plasmaspiegels von etwa 2 ng/ml bis etwa 300 ng/ml ausreicht.

7. Verwendung nach Anspruch 6, worin das aktivierte Protein C angepasst ist zur Verabreichung als eine Bolusinjektion.

8. Verwendung nach Anspruch 6, worin das aktivierte Protein C angepasst ist zur Verabreichung durch eine kontinuierliche Infusion während etwa 1 bis etwa 240 h.

9. Verwendung nach Anspruch 6, worin das aktivierte Protein C angepasst ist zur Verabreichung zuerst als ein Bolus und dann als eine kontinuierliche Infusion.

10. Verwendung nach Anspruch 9, worin ein Drittel des aktivierten Protein C, das zur Bildung eines aktivierten Protein C Plasmaspiegels im Bereich von etwa 2 ng/ml bis etwa 300 ng/ml ausreicht, verabreicht werden soll als eine Bolusinjektion und die verbleibenden zwei Drittel des aktivierten Protein C dann verabreicht werden sollen als eine kontinuierliche Infusion.

## Revendications

1. Utilisation de protéine C dans la préparation d'un médicament pour le traitement d'un patient souffrant de purpura thrombocytopénique thrombotique ou du syndrome urémique hémolytique.

2. Utilisation selon la revendication 1, dans laquelle la protéine C est le zymogène de la protéine C humaine.

3. Utilisation selon la revendication 1, dans laquelle la protéine C est la protéine C humaine activée.

4. Utilisation selon la revendication 3, dans laquelle le médicament est adapté pour une administration en une quantité suffisante de manière à fournir environ 1 µg/kg/heure à environ 50 µg/kg/heure de protéine C humaine activée.

5. Utilisation selon la revendication 4, dans laquelle la protéine C humaine activée est à administrer par infusion continue pendant environ 1 à environ 240 heures.

6. Utilisation selon la revendication 1, dans laquelle la protéine C est la protéine C activée et dans laquelle ledit médicament est adapté pour une administration en une quantité suffisante de manière à fournir un niveau de protéine C activée dans le plasma d'environ 2 ng/ml à environ 300 ng/ml.

7. Utilisation selon la revendication 6, dans laquelle la protéine C activée est adaptée pour une administration en tant qu'injection de bolus.

8. Utilisation selon la revendication 6, dans laquelle la protéine C activée est adaptée pour une administration par une infusion continue pendant environ 1 à environ 240 heures.

9. Utilisation selon la revendication 6, dans laquelle la protéine C activée est adaptée pour une administration, d'abord en tant que bolus, puis en tant qu'infusion continue.

10. Utilisation selon la revendication 9, dans laquelle un tiers de la protéine C activée nécessaire pour atteindre les niveaux de protéine C activée dans le plasma dans la plage d'environ 2 ng/ml à environ 300 ng/ml est à administrer par une injection de bolus et les deux tiers restants de la protéine C activée sont à administrer consécutivement par une infusion continue.
